# EUROPEAN PATENT APPLICATION

(11) **EP 3 909 645 A1**
(43) Date of publication of application: **17.11.2021**
(21) Application number: 20738156.7
(22) Date of filing: 07.01.2020
(51) Int. Cl.: A61P 7/04, A61K 31/655, C07D 409/12

(54) **DOSING REGIMEN OF BICYCLIC SUBSTITUTED PYRAZOLONE AZO DERIVATIVE**

(30) Priority: 08.01.2019 CN 201910016024
(71) Applicant: Jiangsu Hengrui Medicine Co., Ltd., Lianyungang, Jiangsu 222047 (CN)
(72) Inventor: SHEN, Yancong, Lianyungang, Jiangsu 222047 (CN); YANG, Guoping, Lianyungang, Jiangsu 222047 (CN); HUANG, Jie, Lianyungang, Jiangsu 222047 (CN)
(74) Representative: dompatent von Kreisler Selting Werner - Partnerschaft von Patent- und Rechtsanwälten mbB
(86) International application number: PCT/CN2020/070579
(87) International publication number: WO 2020/143593

(57) **Abstract**

A dosing regimen of a bicyclic substituted pyrazolone azo derivative. In particular, the present invention relates to a method for treating a disease, comprisng administering to a patient an effective amount of a compound represented by formula I or a pharmaceutically acceptable salt thereof, wherein the interval between administration and food intake is at least 2 hours.

## Description

The present application claims the priority of Chinese patent application CN201910016024.9 with application date of January 8, 2019. The entire content of the above-mentioned Chinese patent application is incorporated herein by reference.

### Technical Field

The present disclosure belongs to the field of medicine, and relates to a dosing regimen of a bicyclic substituted pyrazoloneazo derivative.

### Background

Thrombopoietin (TPO), also known as megakaryocyte growth and development factor (MGDF), is a 332-amino acid glycosylated polypeptide that plays a key role in processes of regulating megakaryocytopoiesis and the production of platelets from bone marrow megakaryocytes (Kuter et al., Proc.Nat.Acad.Sci.USA 91: 11104-11108 (1994); Barley et al., Cell 77: 1117-1124 (1994); Kausansky et al., Nature 369: 568-571 (1994); Wendling et al., Nature 369: 571-574 (1994); and Sauvage et al., Nature 369: 533-538 (1994)).

Platelets are essential for blood coagulation. When its number is very small, a patient is at risk of bleeding to death. Therefore, TPO has been used to treat a variety of blood diseases.

CN101679286A discloses a series of bicyclic substituted pyrazoloneazo derivatives. Among these, a preferable compound represented by Formula I, is a thrombopoietin (TPO) receptor agonist that can enhance the production of platelets, and is used to treat a variety of blood diseases such as diseases caused by platelet defects. Meanwhile, it also can be used to treat thrombocytopenia, and especially to treat decrease of platelets caused by chemotherapy, radiotherapy and bone marrow transplantation performed for treating cancers and lymphomas. CN102159217A discloses a pharmaceutically acceptable salt of the compound represented by Formula I.

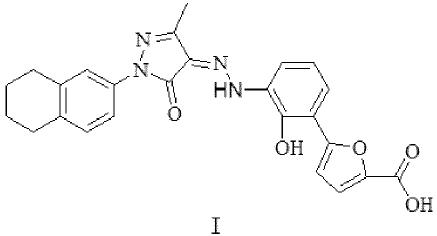

The present disclosure provides an improved dosing regimen of a bicyclic substituted pyrazoloneazo derivative, which can enhance a treatment effect of the medicine.

### Content of the present invention

One aspect of the present disclosure provides a method for treating a disease, including administering an effective amount of a compound represented by Formula I or a pharmaceutically acceptable salt thereof to a patient, wherein an interval between the administration and a meal is at least 2 hours, and preferably the administration is 2 hours before the meal.

In certain implementations, the disease is selected from one or more of thrombocytopenia, thrombocytopenic purpura, and anemia.

In certain implementations, the thrombocytopenia is selected from chemotherapy-induced thrombocytopenia or immune thrombocytopenia. The chemotherapy-induced thrombocytopenia is preferably thrombocytopenia caused by chemotherapy for a malignant tumor. The immune thrombocytopenia is preferably primary immune thrombocytopenia, and may be, for example, chronic primary immune thrombocytopenia.

In certain implementations, the anemia is preferably aplastic anemia, and is more preferably severe aplastic anemia.

In certain implementations, the thrombocytopenic purpura is preferably idiopathic thrombocytopenic purpura, and may be, for example, chronic idiopathic thrombocytopenic purpura.

Another aspect of the present disclosure provides a method for enhancing the production of platelets, including administering an effective amount of a compound represented by Formula I or a pharmaceutically acceptable salt thereof to a patient, wherein an interval between the administration and a meal is at least 2 hours, and preferably the administration is 2 hours before the meal.

Another aspect of the present disclosure provides a method for agonizing a TPO receptor, including administering an effective amount of a compound represented by Formula I or a pharmaceutically acceptable salt thereof to a patient, wherein an interval between administration and meal is at least 2 hours, and preferably administration is 2 hours before meal.

According to the present disclosure, the interval between administration and meal is at least 2 hours, which means that the compound represented by Formula I or a pharmaceutically acceptable salt thereof is administered at least 2 hours before meal or at least 2 hours after meal. For example, the compound represented by Formula I or the pharmaceutically acceptable salt thereof is administered 2 hours before meal or 2 hours after meal.

In certain implementations, a dosage of the compound represented by Formula I or its medicinal salt of the present disclosure is selected from 1 to 20 mg, for example, may be 2.5 mg, 3.75 mg, 5 mg, 7.5 mg, 10 mg, 12.5 mg, or 15 mg.

In certain implementations, the administration frequency of the compound represented by Formula I or its pharmaceutically acceptable salt of the present disclosure is once two days, once a day, twice a day, or three times a day.

In certain implementations, the dosage of the compound represented by Formula I or its pharmaceutically acceptable salt is selected from 2.5 mg, 3.75mg, 5mg, 7.5mg, 10mg, 12.5mg, 15mg, and 15 mg, and the administration frequency is once a day.

The pharmaceutically acceptable salt of the medicine of the present disclosure may be a sodium salt, a lithium salt, a potassium salt, a calcium salt, a magnesium salt, an arginine salt, a lysine salt, a methylamine salt, a dimethylamine salt, a trimethylamine salt, an ethylamine salt, a diethylamine salt, a triethylamine salt, an ethanolamine salt, a piperazine salt, a dibenzylethylene diamine salt, a meglumine salt, a tromethamine salt, a tetramethyl quaternary ammonium salt, a tetraethyl quaternary ammonium salt or a choline salt, preferably is the diethylamine salt, the ethanolamine salt, the choline salt, the piperazine salt, the meglumine salt or the tromethamine salt, more preferably is the ethanolamine salt, and most preferably is a diethanolamine salt.

An administration route of the medicine of the present disclosure may be oral administration, parenteral administration or transdermal administration, wherein the parenteral administration includes but is not limited to an intravenous injection, a subcutaneous injection, and an intramuscular injection.

In certain implementations, the patient of the present disclosure is a human.

In the regimens of the present disclosure, the dosing optionally further includes other components, wherein the other components include but are not limited to a colony stimulating factor, a cytokine, a chemokine, an interleukin or cytokine receptor agonist or antagonist, a soluble receptor, receptor agonist or antagonist antibody, or one or more peptides or small molecular substances that have the same action mechanism as the medicine.

According to the present disclosure, through a relatively longer interval between the administration and a meal, an effect of food on treatment is avoided, thereby ensuring a treatment effect of the medicine.

### Brief description of the drawings

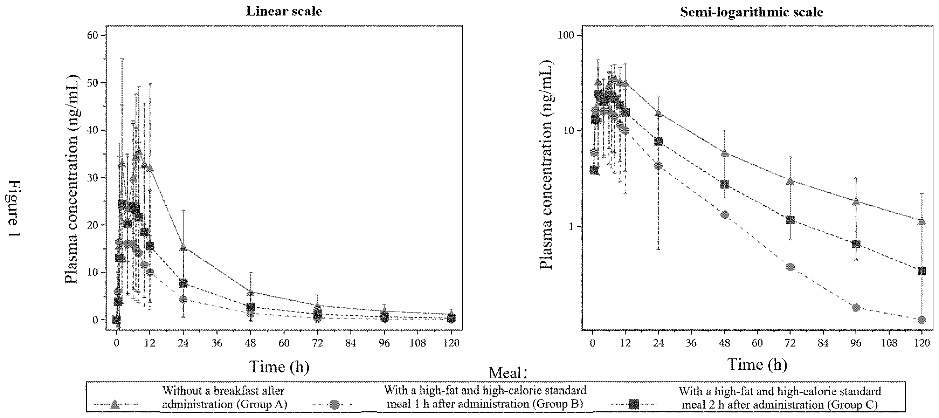

Fig. 1 is a time curve diagram of the mean plasma concentration of a compound represented by Formula I in plasma after a subject orally takes an ethanolamine tablet of the compound represented by Formula I; and

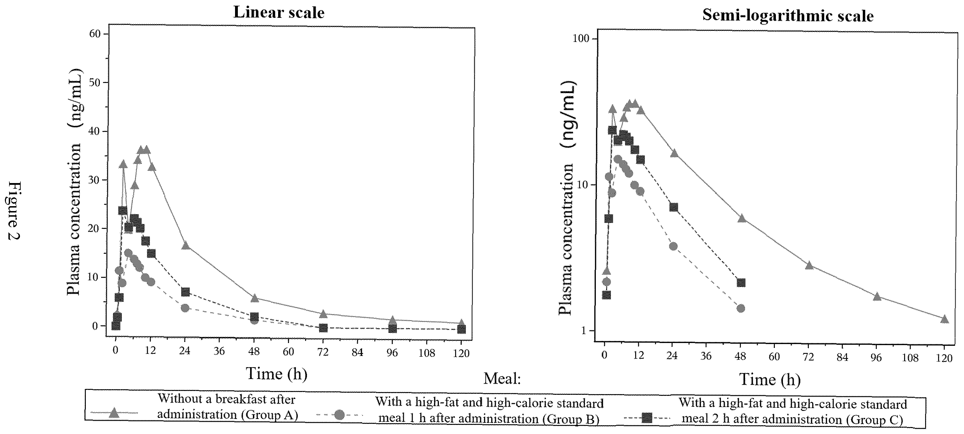

Fig. 2 is a time curve diagram of the median plasma concentration of the compound represented by Formula I.

### Detailed description of the preferred embodiment

Embodiment 1: Study on an effect of different intervals between the administration and a meal on pharmacokinetic parameters of a compound represented by Formula I

### 1. Test product

Diethanolamine salt tablets of the compound represented by Formula I, with specifications of 2.5 mg/table and 5 mg/tablet.

### 2. Dosing regimen

15 healthy adult male subjects were recruited for the trial. They were randomly divided into three trial groups which correspond to three dosing sequences, i.e. ABC, BCA and CAB. A cleaning period is 10 days.

Group A: every subject orally took the test products (one T1: 2.5 mg/tablet and one T2: 5 mg/tablet) once on an empty stomach with 240 mL of room temperature water on the morning of the test. They did not eat breakfast after administration. They were not allowed to drink water within 1 h before administration and 1 h after administration, but were allowed to drink water as needed for the rest time.

Group B: every subject orally took the test products (one T1: 2.5 mg/tablet and one T2: 5 mg/tablet) once on an empty stomach with 240 mL of room temperature water on the morning of the test. They ate high-fat and high-calorie standard meals 1 h after administration, needed to stagger the timing of the breakfast and the blood sampling, and needed to finish the breakfast within 30 min. They were not allowed to drink water within 1 h before administration and 1 h after administration, but were allowed to drink water as needed for the rest time.

Group C: every subject orally took the test products (one T1: 2.5 mg/tablet and one T2: 5 mg/tablet) once on an empty stomach with 240 mL of room temperature water on the morning of the test. They ate high-fat and high-calorie standard meals 2 h after administration, needed to stagger the timing of the breakfast and the blood sampling, and needed to finish the breakfast within 30 min. They were not allowed to drink water within 1 h before administration and 1 h after administration, but were allowed to drink water as needed for the rest time.

The high-fat meal: calories: 800 to 1,000 kcal, composed of 150 kcal of protein, 250 kcal of carbohydrates and 500 to 600 kcal of fats, wherein about 50% of the calories come from the fats. The ingredients and proportions of the standard meals needed to be consistent in each test period. The specific administration sequences are shown in a table below. An effect of food on the medicine was analyzed by sampling blood at fixed time points before and after administration and measuring vital signs.

**Table 1 Administration sequences of the three test groups**

| Administration sequence | First period | Cleaning period | Second period | Cleaning period | Third period |
|---|---|---|---|---|---|
| ABC | A | 10 days | B | 10 days | C |
| BCA | B | [0001] | C | [0001] | A |
| CAB | C | [0001] | A | [0001] | B |

### 3. Test results

In the test, there were Group A (without a breakfast after administration), Group B (with a high-fat and high-calorie standard meal 1 h after administration) and Group C (with a high-fat and high-calorie standard meal 2 h after administration) which correspond to threes administration sequences, i.e. ABC, BCA and CAB. A non-compartmental model (NCA) of WinNonlin 7.0 version was used to calculate PK parameters according to the actual sampling time, and software of SAS 9.4 version was used to list and make descriptive statistical analysis on the PK parameters. Results of descriptive statistical analysis of the PK parameters of the compound represented by Formula I of the subjects in the groups (Group A, Group B and Group C) with different intervals between the administration and a meal are shown in Table 2.

**Table 2 Summary table of the PK parameters of the compound represented by Formula I (PK parameter analytic set)**

| PK parameter (unit) | Statistic | Group A (N=15) | Group B (N=14) | Group C (N=14) |
|---|---|---|---|---|
| Tₘₐₓ (h) | Median (Min, Max) | 7.00 (1.00, 10.00) | 2.00(1.00,6.00) | 5.01 (1.00, 7.00) |
| Cₘₐₓ (ng/mL) | Mean±SD | 39.2461±18.4192 | 21.0785±16.5213 | 28.5985±22.022 1 |
| | Geomean (%CV_{b}) | 35.1224 (55.00) | 15.4008 (107.83) | 19.5670 (132.58) |
| AUCo-t (h^{∗}ng/mL) | Mean±SD | 1040.5777±578.1 595 | 314.2101±289.4848 | 546.7096±504.4 278 |
| | Geomean (%CV_{b}) | 857.0649 (83.81) | 201.9620 (152.17) | 303.1993 (218.68) |
| AUC_{0-∞} (h^{∗}ng/mL ) | Mean±SD | 1110.4290±609.7 280 | 347.0549±308.6317 | 590.9489±529.7 169 |
| | Geomean (%CV_{b}) | 928.9126 (77.20) | 235.3626 (130.87) | 355.2708 (177.52) |
| %AUCₑₓ (%) | Mean±SD | 7.55±5.71 | 13.65±9.42 | 13.90±11.27 |
| | Geomean (%CV_{b}) | 6.62 (47.14) | 11.39 (65.98) | 10.57 (85.87) |
| λ_{z} (1/h) | Mean±SD | 0.0300±0.0178 | 0.0662±0.0288 | 0.0555±0.0335 |
| | Geomean (%CV_{b}) | 0.0263 (53.83) | 0.0600 (51.71) | 0.0459 (74.81) |
| t_{1/2} (h) | Mean±SD | 29.26±12.20 | 13.09±8.16 | 18.58±12.53 |
| | Geomean (%CV_{b}) | 26.40 (53.83) | 11.55 (51.71) | 15.11 (74.81) |
| V_{z}/F (L) | Mean±SD | 330.5633±128.89 44 | 649.3496±507.7649 | 573.9608±435.5 962 |
| | Geomean (%CV_{b}) | 307.5679 (41.69) | 530.9334 (66.66) | 460.2485 (73.41) |
| CL/F (L/h) | Mean±SD | 10.4506±9.4394 | 52.4396±60.4906 | 42.1126±54.2305 |
| | Geomean (%CV_{b}) | 8.0740 (77.20) | 31.8657 (130.87) | 21.1107 (177.52) |
| MRT(h) | Mean±SD | 33.25±8.07 | 18.45±6.79 | 23.13±9.45 |
| | Geomean (%CV_{b}) | 32.15 (28.83) | 17.41 (36.24) | 21.41 (42.66) |

After the subjects orally took 7.5 mg of the ethanolamine tables of the compound represented by Formula I once, the food and the intervals between the administration and the meal affected the PK parameters. A Tₘₐₓ median of Group A (without a breakfast after administration) was 7.00 h; a Tₘₐₓ median of Group B (with a high-fat and high-calorie standard meal 1 h after administration) was 2.00 h; and a Tₘₐₓ median of Group C (with a high-fat and high-calorie standard meal 2 h after administration) was 5.01 h, which indicates that the time-to-peak of the compound represented by Formula I is earlier after a meal, and the shorter an interval between administration and a meal, the more obvious the early trend. Mean Cₘₐₓ of Group A was 39.2461 ng/mL, mean Cₘₐₓ of Group B and mean Cₘₐₓ of Group C were 21.0785 ng/mL and 28.5985 ng/mL, respectively; mean AUCo-t of Group A was 1040.5777 h^{∗}ng/mL, mean AUCo-t of Group B and mean AUCo-t of Group C were 314.2101 h^{∗}ng/mL and 546.7096h^{∗}ng/mL, respectively; mean AUC_{0-∞} of Group A was 1110.4290 h^{∗}ng/mL, and mean AUC_{0-∞} of Group B and mean AUC_{0-∞} of Group C were 347.0549 h^{∗}ng/mL and 590.9489 h^{∗}ng/mL, respectively, which indicates that food reduces the degree of absorption of the compound represented by Formula I, and the change tends to be obvious with the shortening of an interval between administration and a meal. In addition, mean CL/F of Group A was 10.4506 L/h, mean CL/F of Group B and mean CL/F of Group C were 52.4396 L/h and 42.1126 L/h, respectively; mean t_{1/2} of Group A was 29.26 h, and mean t_{1/2} of Group B and mean t_{1/2} of Group C were 13.09 h and 18.58 h, respectively, which indicates that food accelerates the elimination of the compound represented by Formula I, and the change tends to increase with the shortening of an interval between administration and a meal.

A mixed-effects model was used to perform analysis of variance (ANOVA) on the PK parameters (Cₘₐₓ, AUCo-t and AUC_{0-∞}), which are subjected to natural log conversion, of the subjects in the groups with different intervals between administration and a meal by using the software of SAS 9.4 version.

**Table 3: Effect of an interval between administration and a meal on PK parameters of the compound represented by Formula I (PK parameter analytic set)**

| Comparison | PK parameter (unit) | Meal | n | GeoLSM Ratio (%) | 90% CI of Ratio (%) |
|---|---|---|---|---|---|
| Group B vs Cₘₐₓ (ng/mL) | | Group B | 14 | 15.4565 44.01 | (29.83, 64.92) |
| Group A | | | | | |
| | | Group A | 15 | 35.1224 | |
| | AUCo-t (h^{∗}ng/mL) | Group B | 14 | 202.696223.65 | (14.82, 37.74) |
| | | Group A | 15 | 857.0649 | |
| | AUC_{0-∞} (h^{∗}ng/mL) | Group B | 14 | 235.626025.37 | (16.56, 38.86) |
| | | Group A | 15 | 928.9126 | |
| Group B vs Cₘₐₓ (ng/mL) | | Group B | 14 | 15.4565 78.12 | (52.78, 115.63) |
| Group C | | | | | |
| | | Group C | 14 | 19.7860 | |
| | AUCo-t (h^{∗}ng/mL) | Group B | 14 | 202.696266.10 | (41.28, 105.84) |
| | | Group C | 14 | 306.6629 | |
| | AUC_{0-∞} (h^{∗}ng/mL) | Group B | 14 | 235.626065.76 | (42.79, 101.08) |
| | | Group C | 14 | 358.2933 | |
| Group C vs Cₘₐₓ (ng/mL) | | Group C | 14 | 19.7860 56.33 | (38.19, 83.11) |
| Group A | | | | | |
| | | Group A | 15 | 35.1224 | |
| | AUCo-t (h^{∗}ng/mL) | Group C | 14 | 306.662935.78 | (22.42, 57.10) |
| | | Group A | 15 | 857.0649 | |
| | AUC_{0-∞} (h^{∗}ng/mL) | Group C | 14 | 358.2933 38.57 | (25.18, 59.10) |
| | | Group A | 15 | 928.9126 | |

Note: GeoLSM is a geometric least squares mean.

Food and an interval between administration and a meal obviously affect the exposure level of the compound represented by Formula I. Ratios of geometric mean of Cₘₐₓ and AUC_{0-∞} as well as their 90%CI of the subjects who ate food 1 h after orally taking once 7.5 mg of the ethanolamine tablets of the compound represented by Formula I (Group B) to those of the subjects who did not eat food after administration (Group A) were 44.01% (29.83%, 64.92%) and 25.37% (16.56%, 38.86%), respectively, which indicates that Cₘₐₓ and AUC_{0-∞} of the compound represented by Formula I of the subjects who ate food 1 h after administration were respectively reduced by about 56% and 75% relative to those of the subjects who did not eat food after administration. However, ratios of geometric mean of Cₘₐₓ and AUC_{0-∞} as well as their 90%CI of the subjects who ate food 2 h after orally taking once 7.5 mg of the ethanolamine tablets of the compound represented by Formula I (Group C) to those of the subjects who did not eat food after administration (Group A) were 56.33% (38.19%, 83.11%) and 38.57% (25.18%, 59.10%), respectively, which indicates that Cₘₐₓ and AUC_{0-∞} of the compound represented by Formula I of the subjects who ate food 2 h after administration were respectively reduced by about 44% and 61% relative to those of the subjects who did not eat food after administration. In addition, ratios of geometric mean of Cₘₐₓ and AUC_{0-∞} as well as their 90%CI of the subjects who ate food 1 h after administration (Group B) to those of the subjects who ate food 2 h after administration (Group C) were 78.12% (52.78%, 115.63%) and 65.76% (42.79%, 101.08%), which indicates that Cₘₐₓ and AUC_{0-∞} of the compound represented by Formula I of the subjects who ate food 1 h after administration were respectively reduced by about 22% and 34% relative to those of the subjects who ate food 2 h after administration.

Based on above, food reduces the plasma exposure level of the compound represented by Formula I, and the shorter an interval between administration and a meal, the more obvious the reduce degree. Results of the preliminary PK/PD correlation analysis show that the maximum change percentage of platelet count of the subject after single administration has the statistically significant correlation with Cₘₐₓ (a correlation coefficient r=0.84, and p<0.0001). In order to ensure that the expected peak concentration is reached after administration and ensure a platelet-increasing effect, and by combining with the patient's compliance in the actual clinical medication, a recommended administration way is that an interval between administration and a meal is at least 2 h.

While specific embodiments of the present invention are described above, it will be understood by those skilled in the art that these are merely illustrative and that various changes and modifications may be made without departing from the principles and spirit of the present disclosure. Therefore, the scope of protection of the present disclosure shall be defined by the appended claims.

## Claims

1. A method for treating a disease, comprising administering an effective amount of a compound represented by Formula I or a pharmaceutically acceptable salt thereof to a patient, wherein an interval between the administration and a meal is at least 2 hours, and preferably the administration is 2 hours before the meal,

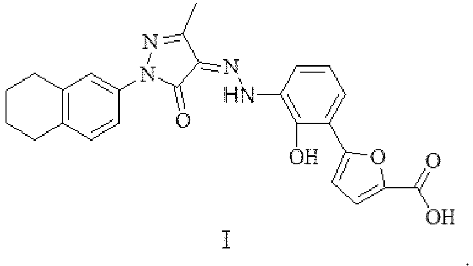

2. The method according to claim 1, wherein the disease is selected from one or more of thrombocytopenia, thrombocytopenic purpura, and anemia, the thrombocytopenia is preferably chemotherapy-induced thrombocytopenia or immune thrombocytopenia; the anemia is preferably aplastic anemia, and the thrombocytopenic purpura is preferably idiopathic thrombocytopenic purpura.

3. A method for enhancing the production of platelets, comprising administering an effective amount of a compound represented by Formula I or a pharmaceutically acceptable salt thereof to a patient, wherein an interval between the administration and a meal is at least 2 hours, and preferably the administration is 2 hours before the meal,

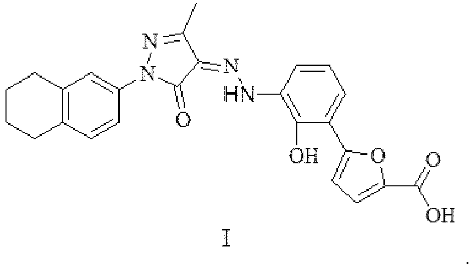

4. A method for agonizing a TPO receptor, comprising administering an effective amount of a compound represented by Formula I or a pharmaceutically acceptable salt thereof to a patient, wherein an interval between the administration and a meal is at least 2 hours, and preferably the administration is 2 hours before the meal,

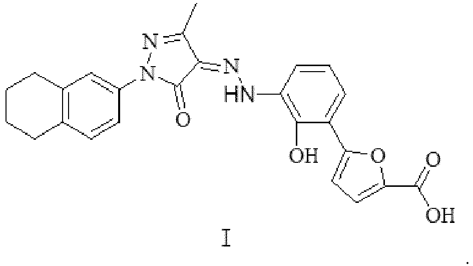

5. The method according to any one of claims 1 to 4, wherein a dosage of the compound represented by Formula I or the pharmaceutically acceptable salt thereof is selected from 1 to 20 mg, and preferably is 2.5 mg, 3.75 mg, 5 mg, 7.5 mg, 10 mg, 12.5 mg, or 15 mg.

6. The method according to any one of claims 1 to 4, wherein the administration frequency of the compound represented by Formula I or the pharmaceutically acceptable salt thereof is once two days, once a day, twice a day, or three times a day.

7. The method according to any one of claims 1 to 4, wherein the pharmaceutically acceptable salt of the compound represented by Formula I is a sodium salt, a lithium salt, a potassium salt, a calcium salt, a magnesium salt, an arginine salt, a lysine salt, a methylamine salt, a dimethylamine salt, a trimethylamine salt, an ethylamine salt, a diethylamine salt, a triethylamine salt, an ethanolamine salt, a piperazine salt, a dibenzylethylene diamine salt, a meglumine salt, a tromethamine salt, a tetramethyl quaternary ammonium salt, a tetraethyl quaternary ammonium salt or a choline salt, preferably is the diethylamine salt, the ethanolamine salt, the choline salt, the piperazine salt, the meglumine salt or the tromethamine salt, more preferably is the ethanolamine salt, and most preferably is a diethanolamine salt.

8. The method according to any one of claims 1 to 4, wherein the administration further includes other components, and the other components include but are not limited to a colony stimulating factor, a cytokine, a chemokine, an interleukin or cytokine receptor agonist or antagonist, a soluble receptor, receptor agonist or antagonist antibody, or one or more of peptides or small molecular substances that have the same action mechanism as the medicine.

9. The method according to any one of claims 1 to 8, wherein the patient is a human.
